# EUROPEAN PATENT APPLICATION

(11) **EP 4 235 226 A1**
(43) Date of publication of application: **30.08.2023**
(21) Application number: 22158514.4
(22) Date of filing: 24.02.2022
(51) Int. Cl.: G01T 1/24

(54) **RADIATION DETECTOR AND DETECTION METHOD**

(71) Applicant: Koninklijke Philips N.V., 5656 AG Eindhoven (NL)
(72) Inventor: JACOBS, Johannes Wilhelmus Maria, Eindhoven (NL); SIMON, Matthias, Eindhoven (NL); WIECZOREK, Herfried Karl, Eindhoven (NL)
(74) Representative: Philips Intellectual Property & Standards

(57) **Abstract**

A radiation detector uses a direct conversion layer (30) with first and second readout sensors located on opposite sides of the direct conversion layer (30). A biasing arrangement (23, 27) provides a voltage bias across the direct conversion layer (30) using pixel electrodes (26) of the first and second readout sensors. The use of a direct conversion layer (30) gives an intrinsic high spatial resolution and enables X-ray photon counting. Two independent readout sensors (e.g. with different technologies and related back-end electronics) are thereby combined in one detector without compromising their functionality. The detector can be made at low cost, by virtue of the use of a single direct conversion layer (30) which is coupled on both sides to multiple readout sensors.

## Description

### FIELD OF THE INVENTION

This invention relates to radiation detectors, for example for use in medical imaging.

### BACKGROUND OF THE INVENTION

X-ray detectors are commonly used for medical diagnostic imaging.

Most current medical X-ray detectors use either the indirect or the direct detection technique. The indirect detection technique uses a scintillator X-ray absorber (usually cesium iodide, CsI) coupled to a matrix of photodiodes such as amorphous silicon photodiodes. These photodiodes also act as storing capacitors. The direct detection technique uses a photoconductor (usually amorphous selenium, a-Se) coupled to an array of storing capacitors. The direct detection technique involves the direct generation of a photocurrent from the incident radiation (without conversion of the X-ray photon to a light photon). New photoconductors with increased X-ray absorption are being developed for use within direct detection X-ray detectors.

Currently, most medical X-ray detectors are based on a single layer of the specific X-ray conversion material (e.g. CsI or a-Se, as explained above) coupled to a single readout sensor fabricated in a specific technology (e.g. a-Si TFT or CMOS). The product characteristics and features are specified within a certain performance range to adequately fulfil imaging tasks for a limited set of clinical applications.

In recent years, there has been an increase in complexity, diversity and requirements of diagnostic and interventional procedures, requiring enhanced detector performance, such as increased dynamic range or spatial resolution, and/or requiring new features such as spectral X-ray imaging. Unfortunately, for most common existing detector designs, only relatively small performance improvements can be realized by optimizing the specifications of the readout sensor and/or the X-ray conversion material. Furthermore, advanced image acquisition methods, signal data electronics, and image processing techniques have their limitations in enhancing imaging performance.

Attempts have been made in the past to increase detector performance and/or functionality by changing the architectural design of the detector.

One proposed design, as described in US9588235, uses a detector which comprises single X-ray scintillator layer, a first readout sensor coupled to its top surface and a second readout sensor coupled to its bottom surface.

Another proposed design, as described in US10371830, has two X-ray scintillator layers stacked on top of each other, with a first readout sensor coupled to the top of the stack and a second readout sensor coupled to the bottom of the stack.

Another proposed design, as described in WO2004095069A1, has a single radiation conversion material for converting X-ray and γ-ray quanta into electrical signals which are amplified and analyzed, for example for generating an X-ray image and a PET image.

Yet another proposed design has a single direct X-ray conversion layer coupled to a single readout sensor capable of simultaneous charge integration and photon counting.

There remains a need for improved detector performance and multi-functionality. Achieving the desired multi-functionality (e.g. spectral X-ray imaging and γ-ray imaging) with an existing X-ray detector design is technically difficult to achieve. For example, starting with a standard design of a charge integration, CI, X-ray detector, it is very difficult to add the new functionality of X-ray photon counting, PC, to this detector, since this would reduce performance of most CI detection modes. Similarly, starting with an existing PC detector design, it is currently technically impossible to modify the design in such a way that CI functionality is added without sacrificing properties of the PC detection mode.

It is also difficult to achieve improved detector performance with a single X-ray conversion layer coupled to a single readout sensor. For example, increasing the spatial resolution by using smaller pixels in the readout sensor would inevitably lead to higher electronic noise. This can be prevented by adding more advanced electronic circuitry to the readout sensor, but at the expense of deteriorating other detector properties, such as dynamic range.

The use of a single detector, and the consequential limited available detection modes, also has implications for the workflow of the physician. For example, the functionality of zooming in to a small Field of View, FOV, area, to investigate small anatomic details at high resolution, is often limited by the spatial resolution of the detector. Also, image acquisitions at higher speed (e.g. > 200 fps for 3D imaging) are often inhibited by the available maximum frame speed (< 100 fps) of the detector. These problems can be resolved by a detector which uses two or more different readout sensors on one side of the x-ray conversion layer, but this has the disadvantage that full-area imaging with one readout sensor type is impossible, and that the detector (or patient) needs to be moved from one imaging position to the other.

It would be desirable to address these issues, but without requiring expensive manufacturing, for example avoiding the need for expensive multi-layer stacked detectors.

### SUMMARY OF THE INVENTION

The invention is defined by the independent claims. The dependent claims define advantageous embodiments.

According to examples in accordance with an aspect of the invention, there is provided a radiation detector comprising:
a direct conversion layer, DCL, having first and second opposing sides;
a first readout sensor located on the first side of the DCL, and comprising first readout sensor pixel electrodes electrically connected to the DCL;
a second readout sensor located on the second side of the DCL and comprising second readout sensor pixel electrodes electrically connected to the DCL; and
a biasing arrangement for providing a voltage bias across the DCL using the first and second pixel electrodes.

The use of a DCL gives an intrinsic high spatial resolution (based on channeling of current in the electric field). This allows a small pixel size to be used for at least one of the first and second readout sensors. The DCL is capable of single X-ray photon counting, using existing large area detectors. Conversely, for commonly used X-ray scintillators, the signal pulse generated by a single X-ray photon is too low in intensity and too slow to be detected by the pixel electronics for photon counting.

The use of DCLs is gaining interest due to the advent of improved photoconductive materials, such as organic-inorganic hybrid perovskites. These materials can be much less expensive than standard direct converter materials such as cadmium telluride, CdTe, or cadmium zinc telluride, CZT, and they perform much better than a-Se.

The detector of the invention offers the possibility to combine two independent readout sensor technologies (and related back-end electronics) in one detector without compromising their functionality. For example, a dedicated charge integration, CI, readout sensor may be coupled to one side of a DCL, and a dedicated photon counting, PC, readout sensor may be coupled to the opposite side of the same DCL. In this way, there are multiple readout sensors in one detector, and each readout sensor can be optimally designed to achieve a required specific performance level within the intended application range of the detector.

The use of multiple readout sensor technologies for example enables a direct zoom-in functionality to be implemented in the detector and the clinical application range is broadened.

The detector can be made at low cost, by virtue of the use of a single DCL which is coupled on both sides to multiple readout sensors. There is also the possibility to cover one side of the DCL only partly with its associated active readout sensor, thereby reducing the costs of the multifunctional detector even further.

The biasing arrangement may be implemented by the readout sensors and their associated readout electronics, or it may be implemented by an additional biasing electrode arrangement.

The detector for example comprises a hybrid detector, wherein the first and second readout sensors have different sensor manufacturing technology and/or radiation detection technology and/or sensor pixel technology. In this way, each readout sensor can be designed for a particular purpose.

The first and second readout sensors for example have different configurations, such as different pixel sizes and/or different readout sensor area. Thus, in addition to different technologies (e.g. semiconductor manufacturing technology such as CMOS vs. TFT, or imaging modality such as charge integration vs. photon counting), different readout sensor pixel layouts and different overall readout sensor areas may be employed.

The first readout sensor for example comprises a charge integration readout sensor, and the second readout sensor comprises a photon counting readout sensor.

In another example, the first and second readout sensors are of the same type (i.e. with the same sensor manufacturing technology, radiation detection technology and sensor pixel technology) but with different configurations, such as different pixel sizes and/or different readout sensor area. Thus, the same type of sensing may be used, but with different configurations, for example to enable different fields of view or different resolutions.

The first readout sensor for example comprises first readout electronics and the second readout sensor comprises second readout electronics, wherein the first and second readout electronics are controllable to read out the first and second readout sensors:
separately;
simultaneously; or
either separately or simultaneously according to setting of the detector.

Thus, the two readout sensors may be used individually and hence in a sequence of operation or simultaneously. The detector may allow the user to choose between these two options as two different modes of operation, or else the detector may only allow one of the modes.

The first and second readout electronics may be controllable to generate said voltage bias between the first and second readout sensors. The readout electronics is for example for charge sensing, and it may hold the pixel electrodes at a reference voltage during the charge sensing. By enabling control of this reference voltage, the desired voltage bias across the DCL may be created.

However, instead of using the readout electronics to implement the desired voltage bias, the first and second readout sensors may each comprise an interconnection grid to enable all readout sensor pixel electrodes to be connected to a reference voltage. In this way, the voltage bias can be applied across the DCL, between the reference voltage of one grid and the operating voltage of the readout electronics of the other readout sensor.

Each interconnection grid for example comprises a respective electrical switch between each pixel electrode and a common reference voltage terminal. This common reference voltage may then be set to a different level to the normal reference voltage (the virtual ground of charge sensitive amplifiers) of the readout electronics, which will be applied when the readout electronics is being used to capture image data. Thus, the common reference voltage applied to the interconnection grid enables a different voltage to be applied to the pixel electrodes than is normally applied by the readout electronics.

According to another aspect of the invention, there is provided a radiation imaging system comprising the radiation detector. The radiation imaging system comprising the detector may advantageously be a system for diagnostic or therapeutic imaging, such as a tomography system or a radiography system or a fluoroscopy system or a combination of such systems.

The invention also provides a radiation detection method comprising:
controlling a biasing arrangement to provide a voltage bias across a DCL using pixel electrodes of first and second readout sensors, wherein the first readout sensor is located on a first side of the DCL and the second readout sensor is located on a second, opposite, side of the DCL;
receiving imaging data from the first readout sensor; and
receiving imaging data from the second readout sensor.

The method is preferably computer-implemented. The method provides a diagnostic image or a pair of diagnostic images for review by a clinician. This invention does not relate to the interpretation of the images for example for the purposes of making a diagnosis.

The method may comprise receiving imaging data from the first and second readout sensors:
separately;
simultaneously; or
selectively either separately or simultaneously.

The method may comprise causing a reference voltage to be applied to the pixel electrodes of one of the first and second readout sensors using an interconnection grid which connects all pixel electrodes of said one of the first and second readout sensors to a reference voltage terminal, and reading out imaging data from the other of the first and second readout sensors.

This approach uses an additional interconnection grid to apply a reference voltage to the pixel electrodes at one side of the DCL so that there is the desired voltage bias relative to the pixel electrode voltages applied by the readout electronics at the other readout sensor.

The method may instead comprise controlling first and second readout electronics of the first and second readout sensors to generate said voltage bias between the first and second readout sensors. This is possible if the voltage bias can be generated between the readout electronics of the two readout sensors. This approach uses the readout electronics to generate the voltage bias.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a schematic cross-section of a standard direct X-ray conversion detector;
Fig. 2 shows an approach disclosed in US 9588235 in which a detector comprises single X-ray scintillator layer and first and second readout sensors on opposite surfaces;
Fig. 3 shows a design in accordance with an example of the invention;
Fig. 4 shows the grid layout used in the design of Figure 3 more clearly;
Fig. 5 shows examples in which both sides of the DCL are fully covered with one or more readout sensors S1 and S2;
Fig. 6 shows examples where the readout sensors have different areas; and
Fig. 7 shows a radiation detection method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a radiation detector which uses a DCL with first and second readout sensors located on opposite sides of the DCL. A biasing arrangement provides a voltage bias across the DCL using pixel electrodes of the first and second readout sensors. The use of a DCL gives an intrinsic high spatial resolution and enables X-ray photon counting. Two independent readout sensors (e.g. with different technologies and related back-end electronics) are thereby combined in one detector without compromising their functionality. The detector can be made at low cost, by virtue of the use of a single DCL which is coupled on both sides to multiple readout sensors.

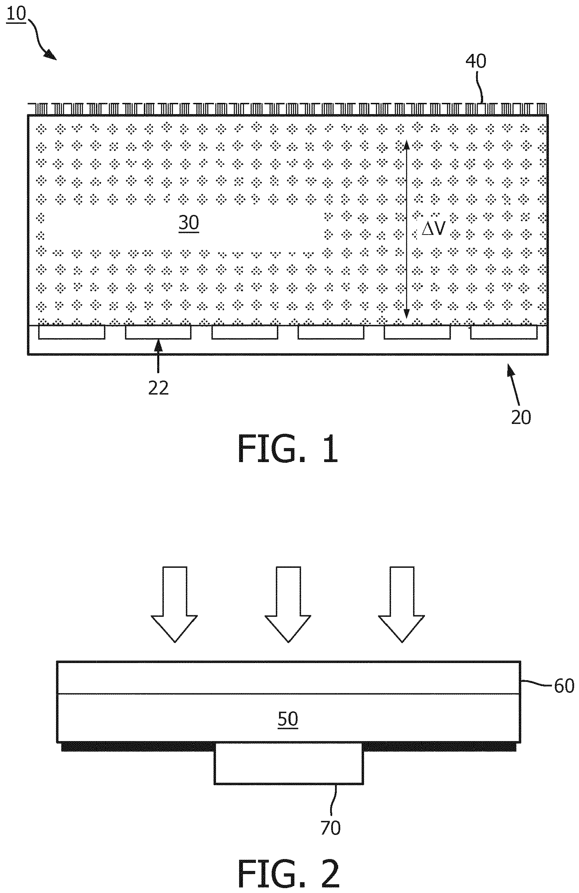

Fig. 1 shows a schematic cross-section of a standard direct X-ray conversion detector 10. A pixelated readout sensor 20 (e.g. a-Si TFT or CMOS) is coupled to one side of a DCL 30 material (e.g. CdTe, CZT, perovskites), and a common bias electrode 40 is connected to the opposite side of the DCL 30.

During radiation exposure of an object, electron-hole pairs are created in the DCL 30. An applied bias electrode voltage ΔV relative to the readout sensor substrate creates an electric field across the DCL 30. This field pulls electrons to the individual readout sensor pixel electrodes 22 and holes to the common bias electrode 40, or vice versa. By accurately collecting and analyzing all signals from the pixel electrodes in the readout sensor 20, a radiation image of the object is generated by additional electronic devices integrated in the readout sensor and/or detector.

It is known to apply different readout sensors to opposite sides of a scintillator layer for an indirect conversion detector. Fig. 2 shows an approach disclosed in US9588235 in which a detector comprises single X-ray scintillator layer 50 (e.g. CsI), a first, thin film transistor, readout sensor 60 coupled to its top surface and a second, CMOS, readout sensor 70 coupled to its bottom surface. The two readout sensors share the same scintillator.

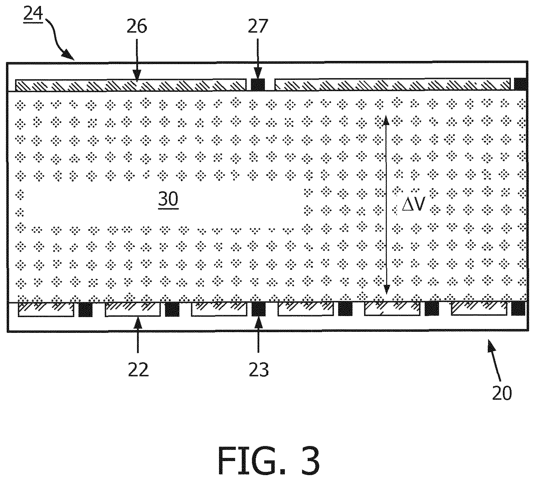

Fig. 3 shows a design in accordance with an example of the invention.

The invention is based on direct conversion, and hence the design of Fig. 3 uses a single DCL 30 as the basic element.

Compared to the standard direct conversion detector of Fig. 1, the common bias electrode is replaced by a second readout sensor 24. In the example shown, the second readout sensor 24 has larger area second pixel electrodes 26, hence a lower spatial resolution, than the first pixel electrodes 22 of the first readout sensor 20. However, this is just one option. The pixel electrodes are electrically connected to the DCL 30.

A voltage bias needs to be generated across the DCL 30.

In the designs in accordance with the invention, this voltage bias is created by a biasing arrangement, which provides the voltage bias across the DCL 30 by using the first and second pixel electrodes 22, 26 (at least for part of the area of the DCL 30 where the first and second readout sensors overlap).

The voltage bias will depend on the material of the DCL 30 and may be around 10V or 100V or more.

There are various ways to implement the voltage bias.

Each readout sensor 20, 24 has readout electronics. The readout electronics is based on current sensing, in particular sensing the current generated in response to the incident radiation, e.g. the incident X-ray photons or gamma radiation. The readout electronics thus typically comprises a charge sensitive amplifier. This amplifier holds the pixel electrode to a reference voltage and measures a charge flowing.

In order to create a bias voltage across the DCL 30 using the pixel electrodes as the voltage bias arrangement, the pixel electrodes of one readout sensor need to be held at a different voltage to the pixel electrodes of the other readout sensor. This may be achieved in various ways.

Fig. 3 shows an example in which each readout sensor comprises a common conductive bias grid, to which all pixel electrodes can be connected via additional electrical switches. The first readout sensor 20 has a first grid 23 and the second readout sensor 24 has a second grid 27. The grids comprise an array of conductors extending between the areas of the pixel electrodes.

The switches are not shown. Additional conductive lines which are needed to address the switches are also not shown.

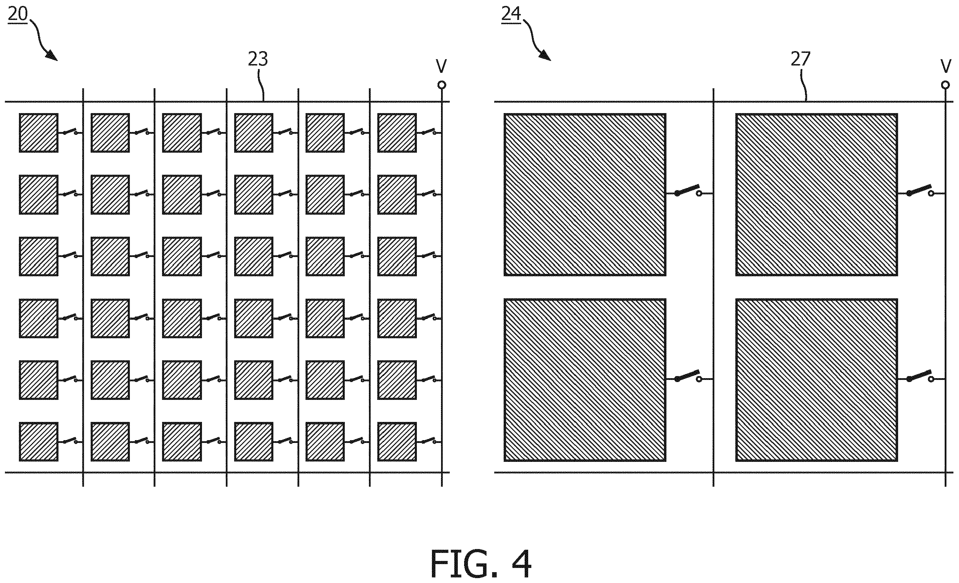

The grid layout is shown more clearly in Fig. 4. The two readout sensors thus share the same DCL 30. During radiation exposure, the required voltage bias ΔV across the DCL 30 can be applied by means of the bias grids 23, 27, and in particular the voltage between one bias grid and the pixel electrodes of the other readout sensor when it is collecting image data.

For example, when the detector is used for the readout of the first readout sensor 20, all pixel electrodes 26 of the opposite second readout sensor 24 are connected via the electrical switches (not shown) to the bias grid lines 27 and hence to a common reference potential V. The voltages at the pixel electrodes of the first readout sensor 20 will be set by the readout electronics of the first readout sensor, i.e. at a virtual ground reference.

Similarly, when the detector is used for the readout of the second readout sensor 24, all pixel electrodes 22 of the first readout sensor 20 are biased by the integrated bias grid in the first readout sensor to the reference potential V. The voltages at the pixel electrodes of the second readout sensor 24 will be set by the readout electronics of the second readout sensor, i.e. at a virtual ground reference.

The inputs of the charge-integrating circuits (in the non-used readout sensor) are held at (or close to) the same potential as the associated bias grid, to avoid any lateral current flow and thereby to save the amplifiers from overload.

An alternative to the use of the bias grid is to use the charge integrating pixel amplifiers themselves all the time. In this case, the bias potentials (i.e. the ground references mentioned above) of the two readout sensors are maintained at different values.

In use, all pixels are used to measure a displacement current in the DCL 30. This current may be measured anywhere in a closed circuit from one side of the detector to the other side, and the measurement is done by integrating the current flow into the pixel electrode. The sum of the currents into all pixels, measured on either detectors side, will therefore be the same.

The charge integration may be measured for large pixels on one detector side and for small pixels on the other detector side. This reduces the number of charge integrating amplifiers, or enables a zoom-in function to be created. Large pixels may for example be used for charge integration on one detector side in combination with small pixels for photon counting on the opposite detector side. This requires different sensor electronics for the two readout sensors.

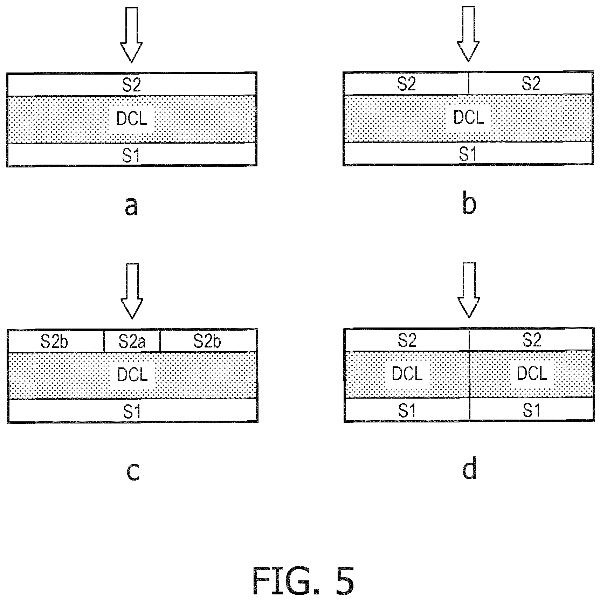

The two readout sensors may have the same overall size or different overall sizes, as well as optionally having pixel electrodes of different sizes (hence different resolutions). Fig. 5 shows various possible examples. In each case, the first readout sensor is denoted by S1 and the second readout sensor is denoted by S2. In the examples of Fig. 5, both sides of the DCL 30 are fully covered with one or more readout sensors S1 and S2.

Fig. 5a shows an example in which both readout sensors are composed from a single large-area substrate (e.g. glass), whereas Figs. 5b to 5d show examples in which one or both readout sensors are formed from multiple tiled small-area substrates (e.g. Si CMOS).

The DCL 30 may also consist of a single piece (Figure 5a to 5c) or it may also be formed from multiple pieces (Fig. 5d).

The complete detector may be assembled from multiple tiled sub-detector modules, as shown schematically in Fig. 5d.

When a readout sensor is formed as a tiled array, there may be different readout sensor designs, such as different readout sensors S2a and S2b in Fig. 5c.

A required specification of the detector performance and/or functionality may be realized, or at least be approached, by proper selection of a combination of two (or more) different readout sensor designs. A non-exhaustive list of possible readout sensor characteristics is given in Table 1. Many combinations of different readout sensor functionalities, specifications, technologies, and/or embedded readout sensor electronics are possible to realize a two-in-one detector which matches closely the increased requirements of the intended imaging applications.

**Table 1**

| **Nr.** | **Detector characteristic** | **Readout sensor 1 (S1)** | **Readout sensor 2 (S2)** |
|---|---|---|---|
| 1 | X-ray detection technology | Charge (energy) integration | Photon counting |
| 2 | Spectral imaging functionality | Non-spectral | Spectral |
| 3 | Type of radiation | X-ray | γ-ray |
| 4 | Clinical application (X-ray) | Mammo (< 40 kV) | General X-ray (> 40 kV) |
| 5 | Frame speed | Low (static) | High (dynamic) |
| 6 | Spatial resolution | Small pixel size | Large pixel size |
| 7 | Dynamic range | Small pixel capacitance | Large pixel capacitance |
| 8 | Pixel amplification circuit | Passive pixel (low SNR) | Active pixel (high SNR) |
| 9 | Readout sensor technology | a-Si, poly-Si, IGZO, or organic TFT | CMOS |
| 10 | Digital output (N=14, 16, 18, ...) | ≤ N bits | > N bits |
| 11 | Photon counting rate | Low | High |

As can be seen from Table 1, the readout sensors may differ by one or more of:
detection modality (e.g. charge integration vs. photon counting);
spectral capability;
radiation type;
intended type of medical imaging;
speed, spatial resolution, dynamic range;
readout electronics characteristics;
readout electronics technology;
output data characteristics.

One implementation of particular interest is a hybrid X-ray detector which is capable of both current integration, CI, and photon counting, PC, imaging. For example, a first conventional CI readout sensor can be used for all usual standard (non-spectral) detector modes and applications, whereas a second dedicated PC readout sensor is used for (new) spectral imaging applications. In this case, a dedicated CI readout sensor is coupled to the bottom of the DCL 30 and a dedicated PC readout sensor is coupled to the top of the DCL 30 (or vice versa).

The pixel size in the PC readout sensor may for example be chosen to be larger (e.g. 300 um, CMOS) than pixel size in the CI readout sensor (e.g. 100 um, IGZO TFT) to enhance spectral imaging performance and reduce manufacturing costs.

When both readout sensors are PC sensors, different pixel sizes may be selected to provide two options of different compromise between spectral resolution and spatial resolution.

When both readout sensors are CI sensors, a standard pixel size (e.g. 100 um) may be used in one readout sensor combined with a small pixel size (e.g. 50 um) in the other readout sensor to provide the zoom-in functionality. Alternatively, a central portion of a readout sensor on one side of the DCL 30 (e.g. readout sensor S2a in Fig. 5c) can provide a special feature (e.g. high resolution, high SNR), whereas the surrounding readout sensors (e.g. readout sensor S2b in Fig. 5c) can provide a standard detector functionality.

The detector may be operated in different modes:
(i) A first mode may use only one of the two readout sensors for imaging, depending on the specific need of the application (spectral, zoom mode, resolution etc.).
(ii) A second mode may use both readout sensors at the same time, if the application benefits from it (e.g. large area overview image, zoomed detail or spectral detail). For this mode, the DCL 30 has to work with a pre-selected bias voltage polarity, which leads to collection of different charge carriers at the two readout sensors. For a given polarity of the bias voltage, one sensor will collect electrons, and the other will collect holes. As different types of charge carrier have in general different lifetimes and mobilities, the sensor electronics will be adapted for the respective carrier type (dependent on the chosen DCL material).

The detector may only have the capability of operating according to the first mode, or else the user may be able to select between both modes if the detector has the option for both modes of operation.

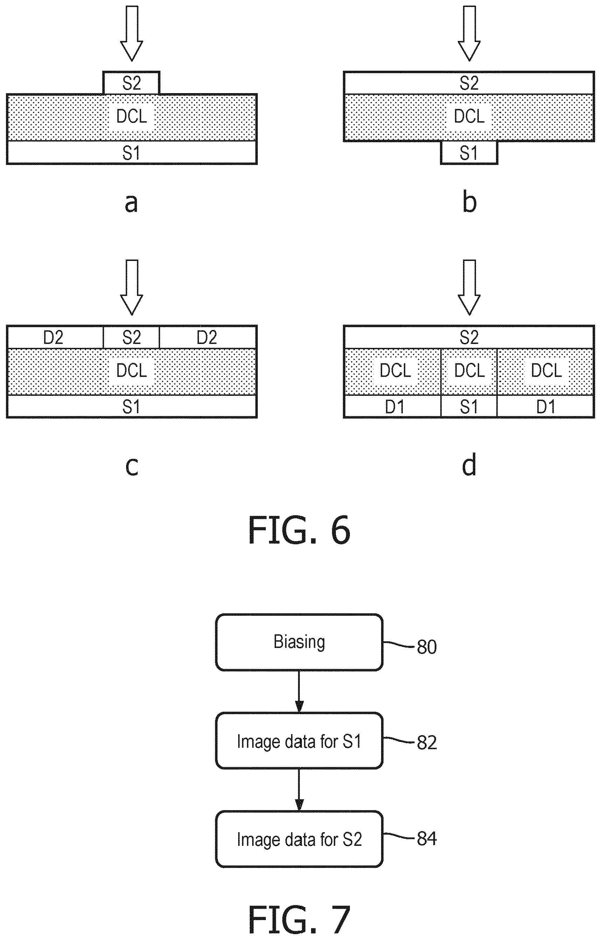

The examples of Fig. 5 show the DCL 30 covered by the first and second readout sensors. However, the readout sensors may have different areas. Some examples are shown in Fig. 6.

In Fig. 6a, the top side of the DCL 30 is covered only partly with the second readout sensor S2. In Figure 6b, the bottom side of the DCL 30 is covered only partly with the first readout sensor S1.

A bias electrode may be provided outside the area of the small readout sensor (S2 in Fig. 6a or S1 in Fig. 6b), because the pixel electrodes of the small readout sensor cannot provide the desired voltage bias across the full area of the DCL 30.

Fig. 6c shows that the smaller readout sensor S2 may be surrounded by a dummy readout sensor D2. Fig. 6d shows that the smaller readout sensor S1 may be surrounded by a dummy readout sensor D1.

The options of Figs. 6a to 6d are interesting when a specific required detector functionality (e.g. spectral photon counting) can be provided in a relatively cheap and simple way by adding a small-area readout sensor to an already existing full-area detector.

Pixels of the dummy readout sensor could in principle be used to provide the voltage bias function without requiring separate bias electrodes as explained above, and without the associated readout electronics.

The detector design should be optimized to ensure that the quality of images acquired by a certain readout sensor are not negatively affected by the presence (or absence) of other readout sensors on the same or opposite side of the DCL 30. For example, due to X-ray absorption in the CMOS Si substrate of a small-area PC readout sensor S2 on top of the DCL 30 (Fig. 6a), the image acquired by the large-area readout sensor S1 at the bottom of the DCL 30 may be slightly disturbed.

The dummy readout sensor mentioned above will enhance the "invisibility" of readout sensor S2 in the image of readout sensor S1, e.g. to avoid disturbing edges in the field of view of readout sensor S1. The non-functional (dummy) readout sensor has the same thickness as the readout sensor it surrounds (PC readout sensor S2 in this example). The dummy readout sensor or sensors D2 can also be used to provide additional metallization lines from the detector periphery to the central PC readout sensor S2, e.g. for power supply and signal data transfer.

Generally, if readout sensors with different thickness (absorption thickness for X-rays, a-Si:H and CMOS detectors) are used, the thinner readout sensor should be on detector side facing the X-ray tube.

Electrical and physical coupling of both readout sensors to the DCL 30 must be adequate to guarantee proper signal transfer from the DCL 30 to all readout sensor pixel electrodes and minimize interference between the two readout sensors.

Well-established interconnect technologies (ACF bonding, bump bonding, soldering, etc.) exist for coupling pixelated readout sensors to various DCL materials. When a high voltage is applied via the bias grid on pixel electrodes of the readout sensors on one side of the DCL 30, it may be needed to protect the electronic circuitry of these temporarily inactive readout sensor pixels. This can be done by temporarily disconnecting all pixel circuits from the bias grid and the pixel electrodes by using an additional electrical switch per pixel.

When a grid is used, preferably the conductive bias grid lines and electrical switches are realized at the very end of the photolithography-based processes which are commonly used to manufacture the readout sensors themselves (e.g. a-Si TFT or Si CMOS). Grid lines may consist of metals or metal alloys (Cu, Al, Mo, W, Cr, Ti, etc.). Alternatively, non-photolithographic methods (3D metal printing, laser-induced metal deposition, screen-printing conductive inks, etc.) can be used to realize a bias grid. Electrical switches may be simple MOSFETs (CMOS) or TFTs (e.g. a-Si or IGZO).

The invention can be applied to a large variety of commonly known DCL materials (e.g. amorphous Se, single crystal Si, single crystal GaAs, polycrystalline or single crystal CdTe and CZT). In particular, the use of polycrystalline or single crystal metal halide perovskite materials (MAPbI3, MAPbBr3) is attractive, since they need a smaller operating voltage (ca. 10 V) than the more known DCL materials (> 100V).

Fig. 7 shows a radiation detection method comprising:
in step 80, controlling a biasing arrangement to provide a voltage bias across the DCL 30 using the pixel electrodes of the first and second readout sensors;
in step 82, receiving imaging data from the first readout sensor; and
in step 84 receiving imaging data from the second readout sensor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Measures recited in mutually different dependent claims may be advantageously combined.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A radiation detector comprising:
a direct conversion layer (30) having first and second opposing sides;
a first readout sensor (20) located on the first side of the direct conversion layer (30), and comprising first readout sensor pixel electrodes (22) electrically connected to the direct conversion layer (30);
a second readout sensor (24) located on the second side of the direct conversion layer (30) and comprising second readout sensor pixel electrodes (26) electrically connected to the direct conversion layer (30); and
a biasing arrangement (23,27) for providing a voltage bias across the direct conversion layer (30) using the first and second pixel electrodes.

2. The detector of claim 1 comprising a hybrid detector, wherein the first and second readout sensors differ in respect of one or more of:
the sensor manufacturing technology;
the radiation detection technology;
the sensor pixel technology.

3. The detector of claim 2, wherein the first and second readout sensors have different configurations, such as different pixel sizes and/or different readout sensor area.

4. The detector of claim 2 or 3, wherein the first readout sensor comprises a charge integration readout sensor, and the second readout sensor comprises a photon counting readout sensor.

5. The detector of claim 1, wherein the first and second readout sensors have the same sensor manufacturing technology, radiation detection technology and sensor pixel technology but have different geometric configurations, such as different pixel sizes and/or different readout sensor area.

6. The detector of any one of claims 1 to 5, wherein the first readout sensor comprises first readout electronics and the second readout sensor comprises second readout electronics, wherein the first and second readout electronics are controllable to read out the first and second readout sensors:
separately;
simultaneously; or
either separately or simultaneously according to setting of the detector.

7. The detector of claim 6, wherein the first and second readout electronics are controllable to generate said voltage bias between the first and second readout sensors.

8. The detector of any one of claims 1 to 6, wherein the first and second readout sensors each comprise an interconnection grid (26, 27) to enable all readout sensor pixel electrodes to be connected to a reference voltage.

9. The detector of claim 8, wherein each interconnection grid comprises a respective electrical switch between each pixel electrode and a common reference voltage terminal.

10. A radiation imaging system comprising the detector according to any of the preceding claims, wherein the radiation imaging system is a tomography system or a radiography system or a fluoroscopy system.

11. A radiation detection method comprising:
(80) controlling a biasing arrangement to provide a voltage bias across a direct conversion layer using pixel electrodes of first and second readout sensors, wherein the first readout sensor is located on a first side of the direct conversion layer and the second readout sensor is located on a second, opposite, side of the direct conversion layer;
(82) receiving imaging data from the first readout sensor; and
(84) receiving imaging data from the second readout sensor.

12. The method of claim 11, comprising receiving imaging data from the first and second readout sensors:
separately;
simultaneously; or
selectively either separately or simultaneously.

13. The method of claim 11 or 12, comprising causing a reference voltage to be applied to the pixel electrodes of one of the first and second readout sensors using an interconnection grid which connects all readout sensor pixel electrodes of said one of the first and second readout sensors to a reference voltage terminal, and reading out imaging data from the other of the first and second readout sensors.

14. The method of claim 11 or 12, comprising controlling first and second readout electronics of the first and second readout sensors to generate said voltage bias between the first and second readout sensors.

15. A computer program comprising computer program code means which is adapted, when said program is run on a computer, to implement the method of any one of claims 11 to 14.
